# EUROPEAN PATENT APPLICATION

(11) **EP 4 353 817 A1**
(43) Date of publication of application: **17.04.2024**
(21) Application number: 22820328.7
(22) Date of filing: 09.06.2022
(51) Int. Cl.: C12N 5/077

(54) **LOW TEMPERATURE-MANAGED CELL AGGREGATES AND CELL AGGREGATE MAINTAINING METHOD**

(30) Priority: 11.06.2021 JP 2021098281
(71) Applicant: Kyoto University, Kyoto-shi, Kyoto 606-8501 (JP); Rege Nephro Co., Ltd., Kyoto-shi, Kyoto, 606-8501 (JP); Nikkiso Co., Ltd., Tokyo 150-6022 (JP)
(72) Inventor: OSAFUNE, Kenji, Kyoto-shi, Kyoto 606-8501 (JP); SUGIMOTO, Shunjiro, Kyoto-shi, Kyoto 606-8501 (JP); RYOSAKA, Makoto, Kyoto-shi, Kyoto 606-8501 (JP); JIMBO, Yoichi, Kanazawa-shi, Ishikawa 920-0177 (JP); KITAGAWA, Fumihiko, Kanazawa-shi, Ishikawa 920-0177 (JP)
(74) Representative: Hoffmann Eitle
(86) International application number: PCT/JP2022/023371
(87) International publication number: WO 2022/260149

(57) **Abstract**

There are provided a cell aggregate that can be used as a cell preparation in regenerative medicine, maintains the original characteristics of cells, and has high safety, and a method for simply and safely maintaining the cell aggregate size, the cell characteristics and viability suitable for use as a cell preparation in regenerative medicine. According to an aspect of the present invention, a cell aggregate maintaining method is a method including a maintenance process of maintaining a liquid containing a plurality of the cell aggregates at a temperature which is lower than a culture temperature of the cells and at which the liquid is not frozen.

## Description

### Technical Field

The present invention relates to a cell aggregate of cultured cells and a cell aggregate maintaining method. The present invention further relates to a cell aggregate producing method and a cell preparation containing cell aggregates.

### Background Art

In recent years, attempts have been made to transplant cells such as stem cells and progenitor cells for repair of damaged tissues and the like. Since these cells have the ability to differentiate into various cells, regenerative medicine using these cells is expected to be a new method for treating diseases that have been difficult to treat. For example, the nephron progenitor cells are embryonic progenitor cells that derive a plurality of epithelial cells constituting renal glomeruli and renal tubules, and it is reported that transplantation of human iPS cell-derived nephron progenitor cell aggregates under the renal capsule alleviates kidney injury in acute kidney injury (AKI) model mice due to ischemia-reperfusion injury (Toyohara T. et al., Stem Cells Transl Med. 2015 Sep; 4(9): 980-992; Hoshina A. et al., Sci Rep. 2019 Jul 18; 9(1): 10701), and transplantation of mouse embryo-derived nephron progenitor cell aggregates under the renal capsule alleviates cisplatin-induced kidney injury in drug-induced AKI model mice (Li Z. et al., Cell Stem Cell. 2016 Oct 06; 19(4): 516-529). Therefore, nephron progenitor cell transplantation therapy is expected as a treatment method for kidney disease.

In order to put such a new treating method based on regenerative medicine into practical use, a method capable of stably producing a cell preparation and a method of maintaining the produced cell preparation in a good state until the time of use are required together with establishment of a treatment method.

In the case of using a cell aggregate cultured as a cell preparation, in the process of culturing, collecting, concentrating, and transporting cells, under a situation where a plurality of cell aggregates are in contact with each other, there is a problem that the cell aggregates adhere to each other until the time of use and become large. When the cell aggregate becomes large, cells inside the aggregate die and the viability decreases. In addition, it is considered that there is a possibility that the cell aggregate is denatured by adhesion, or when the aggregate is larger than the inner diameter of the needle of the cell transplantation device at the time of transplantation, the aggregate may break apart and the therapeutic effect be reduced.

Therefore, in order to realize cell therapy using cell aggregates, it is essential to develop a method in which the cell aggregates do not adhere to each other during the production or transportation of the cell preparation, and the size of the aggregates suitable for transplantation and the properties of the cells that bring about a therapeutic effect are maintained.

Patent Literature 1 describes that adhesion between pluripotent stem cell aggregates can be inhibited by culturing pluripotent stem cells in suspension in a culture medium containing a cell cycle inhibitor such as colchicine or cytochalasin.

However, a cell cycle inhibitor is a drug that stops a cell cycle, which is a cell division cycle, at a specific time and inhibits cell division, and is a substance having strong toxicity to cells. Therefore, in regenerative medicine and the like, when the produced cells are transplanted into a living body, it is necessary to remove the cell cycle inhibitor. The cell cycle inhibitor in the culture solution or the buffer solution can be removed in the washing process, but it is difficult to remove the part taken up by the cells, and there is a concern about an adverse effect on the cell aggregate used in the field of regenerative medicine and the like.

### Citation List

### Patent Literature

Patent Literature 1: International Publication No. WO 2019/131940

### Summary of Invention

### Technical Problem

An object of the present invention is to provide a cell aggregate that can be used as a cell preparation in regenerative medicine, maintains the original characteristics of cells, and has high safety, and a method for simply and safely maintaining the cell aggregate size, the cell characteristics and viability suitable for use as a cell preparation in regenerative medicine.

### Solution to Problem

According to one embodiment of the present invention, there is provided a cell aggregate maintaining method, including: a maintenance process of maintaining a liquid containing a plurality of the cell aggregates at a temperature which is lower than a culture temperature of the cells and at which the liquid is not frozen.

According to another embodiment of the present invention, there is provided a cell aggregate which is produced by a method including: a culturing process of culturing cells in a culture medium to form a plurality of cell aggregates; and a cooling process of cooling a liquid containing a plurality of the cell aggregates to a temperature which is lower than a culture temperature of the cells and at which the liquid is not frozen.

### Advantageous Effects of Invention

According to the present invention, it is possible to prevent adhesion and enlargement of aggregates without using an additive that may adversely affect cells while maintaining the therapeutic effect of cell aggregates such as nephron progenitor cells.

In addition, according to the present invention, it is possible to produce and transport cell aggregates and a cell preparation that maintain the appropriate cell aggregate size and the cell characteristics and viability, and maintain a therapeutic effect until the time of use.

### Brief Description of Drawings

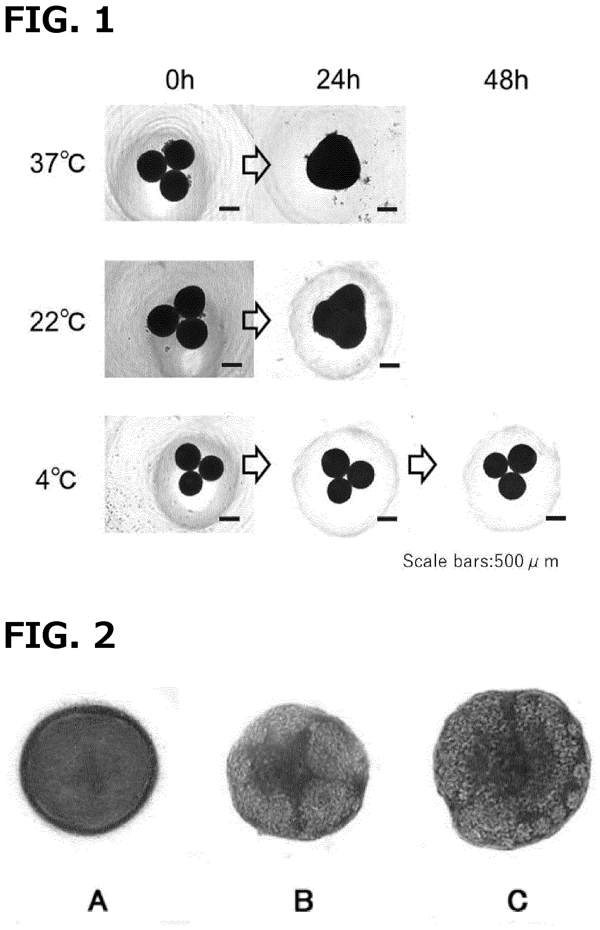

Fig. 1 is a photomicrograph showing a morphology of a plurality of mouse embryo-derived nephron progenitor cell aggregates maintained in contact with each other at various temperatures for a predetermined time. The length of the scale bar in the drawing is 500 µm.
Fig. 2 is a photomicrograph showing the morphology of a mouse embryo-derived nephron progenitor cell aggregate before and after nephron induction. A is a morphology before the nephron induction of the mouse embryo-derived nephron progenitor cell aggregate maintained at 4°C for 48 hours, B is a morphology after the nephron induction of the mouse embryo-derived nephron progenitor cell aggregate maintained at 4°C for 48 hours, and C is a morphology after the nephron induction of the mouse embryo-derived nephron progenitor cell aggregate obtained by the normal method (37°C).

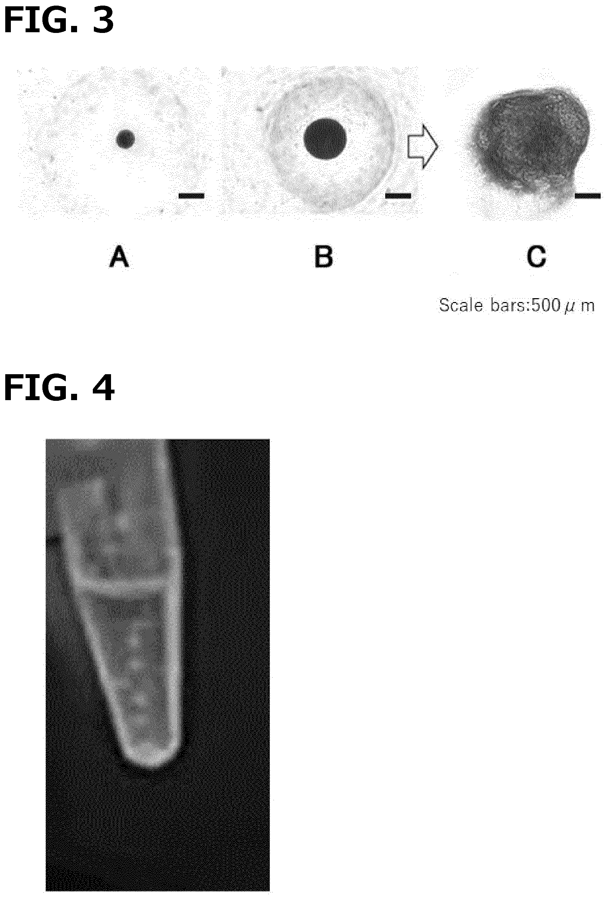

Fig. 3 is a photomicrograph showing the differentiation potency and proliferation potency of mouse embryo-derived nephron progenitor cells maintained at low temperature. The length of the scale bar in the drawing is 500 µm. A is a morphology of the mouse embryo-derived nephron progenitor cells that were maintained at 4°C for 48 hours, then repassaged, and cultured at 4°C for 144 hours, B is a morphology of the mouse embryo-derived nephron progenitor cells that were maintained at 4°C for 48 hours, then repassaged, and cultured at 37°C for 144 hours before nephron induction, and C is a morphology of the mouse embryo-derived nephron progenitor cells that were maintained at 4°C for 48 hours, then repassaged, and cultured at 37°C for 144 hours after nephron induction.
Fig. 4 is a photograph showing a state immediately after human iPS cell-derived nephron progenitor cell aggregates are maintained in a 1.5 mL tube at 4°C for 24 hours and the culture solution is stirred.

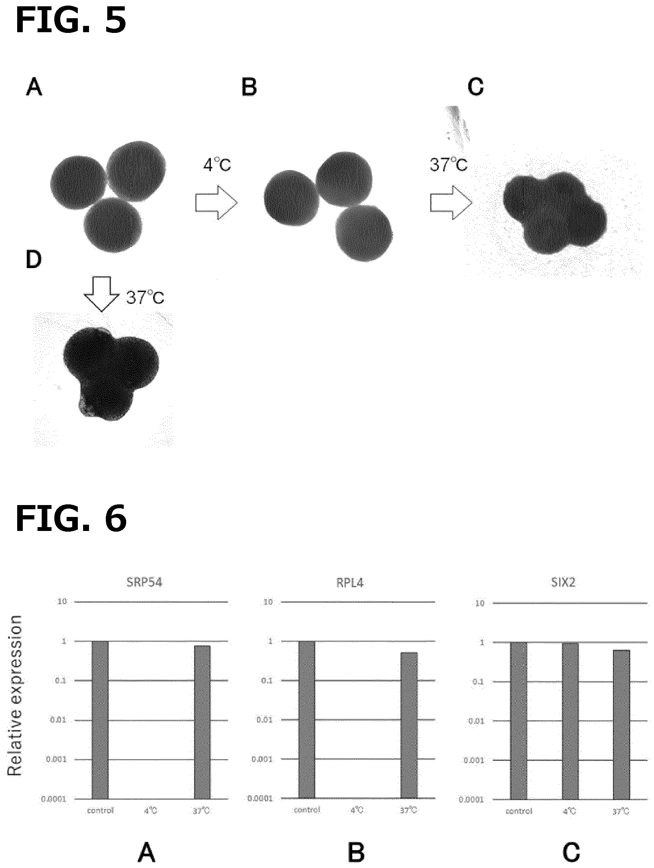

Fig. 5 is a photomicrograph showing a morphology of a plurality of human iPS cell-derived nephron progenitor cell aggregates maintained in contact with each other at various temperatures for a predetermined time. A is a morphology before maintenance, B is a morphology after being left to stand at 4°C for 24 hours, C is a morphology after being left to stand at 4°C for 24 hours, further being left to stand at 37°C for 24 hours, and D is a morphology after being left to stand at 37°C for 24 hours.
Fig. 6 is a diagram showing the influence of low-temperature storage on the expression levels of a protein transport-related marker gene (SRP54), a protein synthesis-related marker gene (RPL4), and a nephron progenitor cell -related marker gene (SIX2) in human iPS cell-derived nephron progenitor cell aggregates. A is a diagram for comparing the expression levels of SRP54, B is a diagram for comparing the expression levels of RPL4, and C is a diagram for comparing the expression levels of SIX2, respectively, under three conditions of before maintaining the low temperature ("control"), after being left to stand at 4°C for 24 hours ("4°C"), after being left to stand at 4°C for 24 hours, and after being left to further stand at 37°C for 24 hours ("37°C").

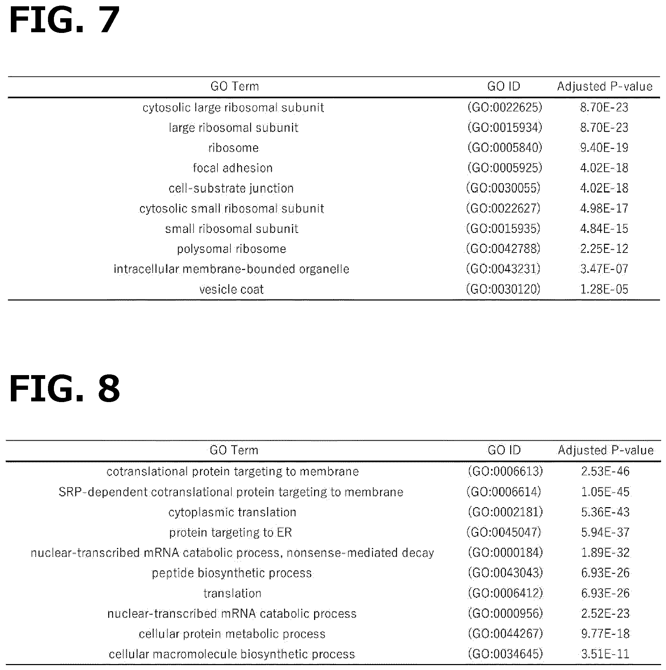

Fig. 7 is a table showing GO terms, GO ID, and Adjusted P-values of the top 10 gene groups in which the gene expression level was decreased in the sample left to stand at 4°C for 48 hours, compared with the sample of the human iPS cell-derived nephron progenitor cell aggregates left to stand at 37°C for 48 hours, among the gene groups related to the components of the cell.
Fig. 8 is a table showing GO terms, GO ID, and Adjusted P-values of the top 10 gene groups in which the gene expression level was decreased in the sample left to stand at 4°C for 48 hours, compared with the sample of the human iPS cell-derived nephron progenitor cell aggregates left to stand at 37°C for 48 hours, among the gene groups related to the biological process.

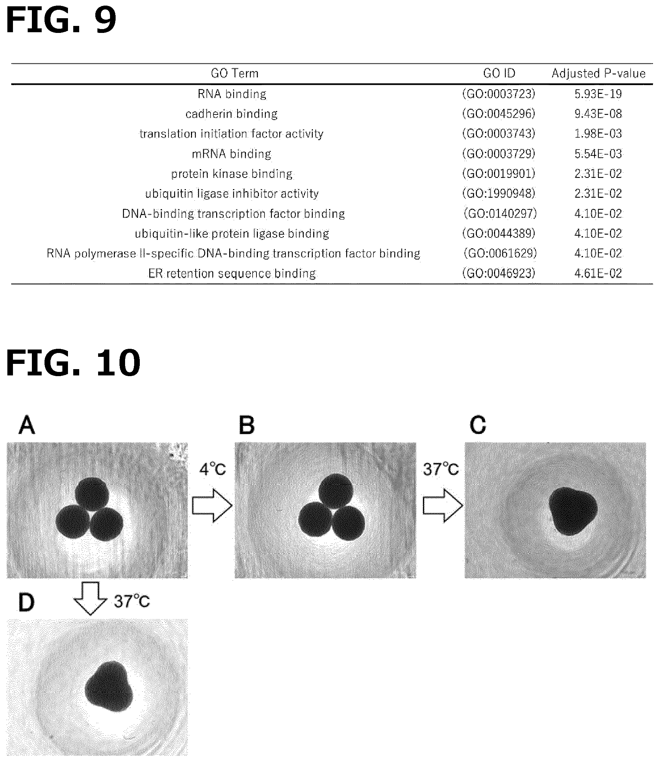

Fig. 9 is a table showing GO terms, GO ID, and Adjusted P-values of the top 10 gene groups in which the gene expression level was decreased in the sample left to stand at 4°C for 48 hours, compared with the sample of the human iPS cell-derived nephron progenitor cell aggregates left to stand at 37°C for 48 hours, among the gene groups related to the molecular function.
Fig. 10 is a photomicrograph showing a morphology of a plurality of human iPS cell aggregates maintained in contact with each other at various temperatures for a predetermined time. A is a morphology before maintenance, B is a morphology after being left to stand at 4°C for 24 hours, C is a morphology after being left to stand at 4°C for 24 hours, and further being left to stand at 37°C for 24 hours, and D is a morphology after being left to stand at 37°C for 24 hours.

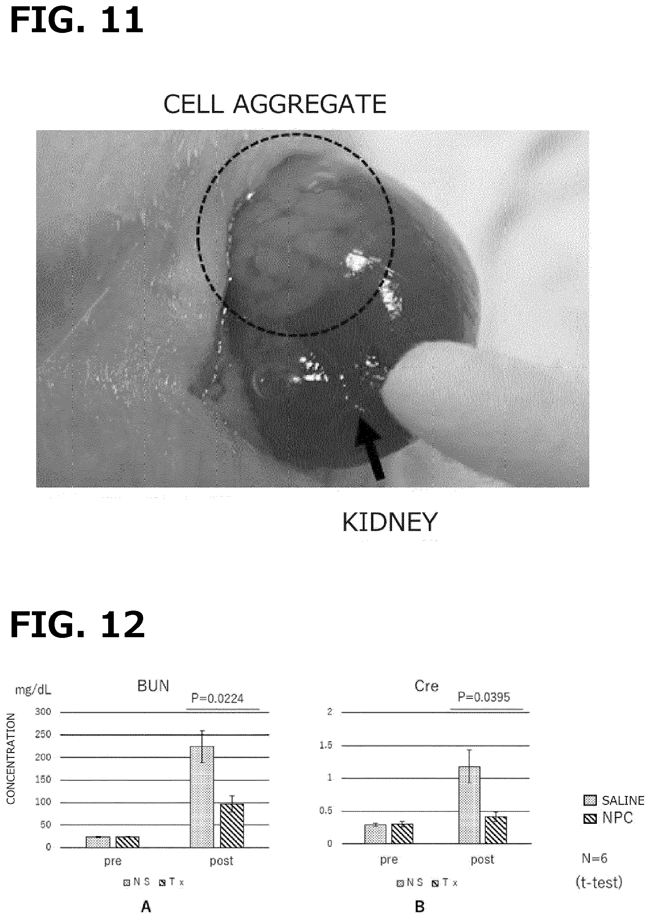

Fig. 11 is a photograph showing a kidney of a cisplatin-induced acute kidney injury model mouse and human iPS cell-derived nephron progenitor cell aggregates transplanted under the renal capsule.
Fig. 12 is a diagram showing an effect of transplantation of human iPS cell-derived nephron progenitor cell aggregates under the renal capsule of a cisplatin-induced acute kidney injury model mouse. A is a diagram showing a blood urea nitrogen ("BUN") value which is a mouse renal function marker, and B is a diagram showing a serum creatinine ("Cre") concentration. In the human iPS cell-derived nephron progenitor cell aggregates ("NPC") or physiological saline ("saline"), "pre" represents a value before administration, and "post" represents a value on Day 4 after administration.

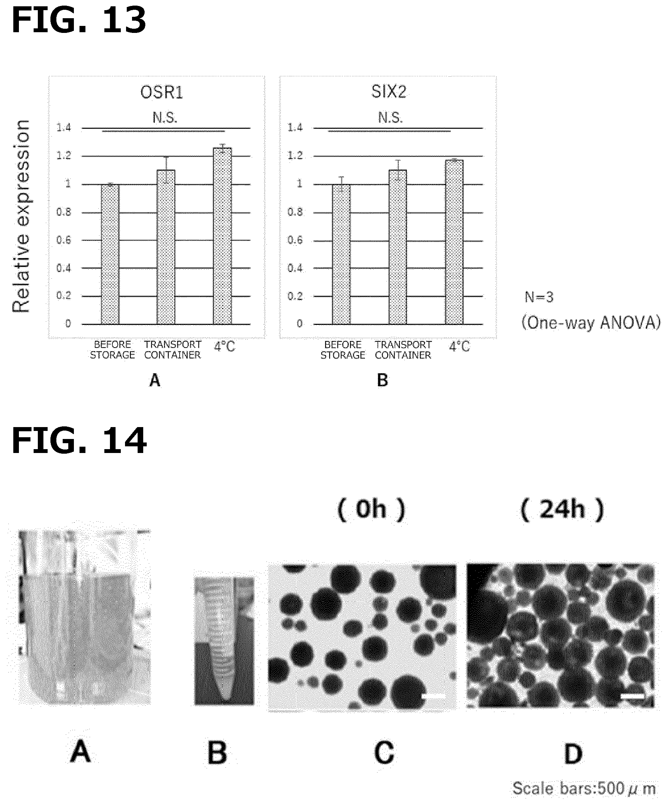

Fig. 13 is a diagram showing the influence of low-temperature storage on the expression level of a nephron progenitor cell -related marker gene (OSR1, SIX2) in human iPS cell-derived nephron progenitor cell aggregates. A is a diagram for comparing the expression levels of OSR1, and B is a diagram for comparing the expression levels of SIX2, respectively, under three conditions of before low-temperature storage ("before storage"), after storage for 48 hours in a storage container of a transport system (5°C to 6°C) ("transport container"), and after storage for 48 hours in a refrigerator (2°C to 4°C) ("4°C").
Fig. 14 is a photograph showing inhibition of adhesion of mouse embryo-derived nephron progenitor cells obtained in a bioreactor. The length of the scale bar in the drawing is 500 µm. A indicates a mouse embryo-derived nephron progenitor cell aggregates in a bioreactor immediately after completion of culture, and B indicates a mouse embryo-derived nephron progenitor cell aggregates in a state of being transferred to a 15 mL tube and naturally settled. C is a photomicrograph of mouse embryo-derived nephron progenitor cell aggregates immediately after completion of culture, and D is a photomicrograph of mouse embryo-derived nephron progenitor cell aggregates after maintaining at 4°C for 24 hours.

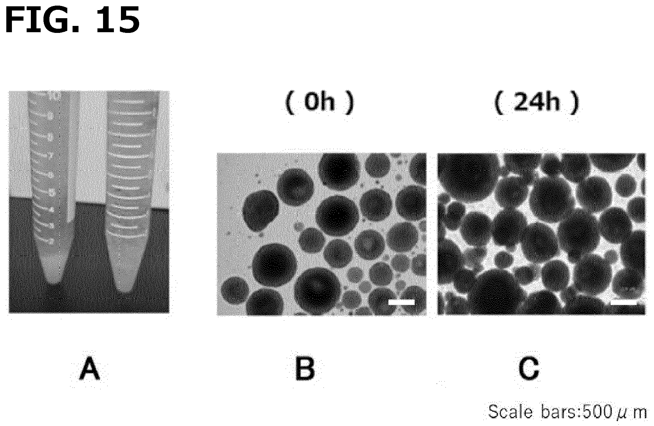

Fig. 15 is a photograph showing the effect of addition of methylcellulose. The length of the scale bar in the drawing is 500 µm. A is a photograph showing the state of mouse embryo-derived nephron progenitor cell aggregates in a normal medium (left) and a medium containing 0.6% methylcellulose (right). B is a photomicrograph of mouse embryo-derived nephron progenitor cell aggregates immediately after the completion of culture in a normal medium, and C is a photomicrograph of mouse embryo-derived nephron progenitor cell aggregates after maintaining at 4°C for 24 hours in a medium containing 0.6% methylcellulose.

### Description of Embodiments

Hereinafter, the present invention will be described in detail. In the present specification, the notation "a to b" in the description of the numerical range represents a or more and b or less unless otherwise specified. In addition, when a plurality of upper limit values and a plurality of lower limit values are listed separately, it is assumed that all numerical ranges that can be set by freely combining these upper limit values and lower limit values are described.

According to one embodiment of the present invention, there is provided a cell aggregate maintaining method, including: a maintenance process of maintaining a liquid containing a plurality of the cell aggregates at a temperature which is lower than a culture temperature of the cells and at which the liquid is not frozen. In addition, according to another embodiment, a cell aggregate producing method includes: a culturing process of culturing cells in a culture medium to form a plurality of cell aggregates; and a cooling process of cooling a liquid containing a plurality of the cell aggregates to a temperature which is lower than a culture temperature of the cells and at which the liquid is not frozen.

The cells used in the present invention are not particularly limited as long as the cells form an aggregate in a culture solution. The cell is preferably derived from a mammal, and is preferably derived from a species generally used for studies of primates such as humans and monkeys or mice. Examples of cells include cells used for research on regenerative medicine, cells used as cell preparations, and for example, undifferentiated cells. The undifferentiated cell refers to a cell having self-replication potency and differentiation potency. Specific examples of the undifferentiated cells include pluripotent stem cells such as ES cells and iPS cells, various progenitor cells such as nephron progenitor cells, ureteric bud cells, and stromal precursor cells, and various stem cells such as mesenchymal stem cells, neural stem cells, and adipose stem cells. The cell may be a cell induced to be differentiated from an undifferentiated cell, an immortalized cell, or an established cell, or may be a primary cultured cell isolated from a tissue. In addition, depending on the purpose, the cell may be a normal cell, or a cell having a disease, a genetic abnormality, or a transgene.

The cells can be cultured according to a conventional method, for example, under conditions of 37°C and 5% CO₂ by using a medium and a culture vessel suitable for the cells to be cultured. The medium for culturing cells is not particularly limited, and an appropriate medium can be selected and used depending on the cells. In addition, conventionally known materials and additives useful for cell culture can be appropriately used.

The culture may be any of static culture, shaking culture, stirring culture, and the like. The culture may be adhesion culture, but it is preferable to perform culture in a non-adhesion state (for example, suspension culture) for at least a part of the period. Certain cells can form cell aggregates by culturing in a non-adherent state in a culture vessel. The "non-adhered state" refers to a state where all or most of the cells are not adhered to the surface of the culture vessel, and includes a state where all or most of the cells are separated from the surface of the culture vessel and a state where even when the cells are in contact with the surface of the culture vessel, the cells can be easily separated from the surface of the culture vessel by coating of the culture vessel, convection of the culture medium, or the like without using a tool, an enzyme, or the like.

Any culture vessel can be used. A dish or plate subjected to a cell-free (low) adhesion treatment for facilitating the formation of aggregates may be used, and a spinner flask or a bioreactor may be used for mass culture.

As used herein, the term "aggregate" of cells refers to a massive aggregate of several or more cells. The number of cells constituting the aggregate can be any number depending on the type of cells, intended use, and the like, and is, for example, 1,000 or more, 5,000 or more, 10,000 or more, 100,000 or more, or 200,000 or more. The number of cells constituting the aggregate is, for example, 500,000 or less, 400,000 or less, 300,000 or less, or 200,000 or less. The number of cells of the aggregate having a predetermined diameter can be calculated by measuring the diameter (definitions and measurement methods will be described later.) and the number of cells of the prepared aggregate in advance. In the case of a nephron progenitor cell aggregate, an aggregate having a diameter of 200 µm is formed with the number of cells of about 5,000 to about 10,000, an aggregate having a diameter of 500 µm is formed with the number of cells of about 50,000 to about 100,000, and an aggregate having a diameter of 800 µm is formed with the number of cells of about 100,000 to 300,000. Therefore, the number of cells constituting the nephron progenitor cell aggregate is preferably 5,000 or more, more preferably 10,000 or more, and most preferably 50,000 or more, and is preferably 500,000 or less, more preferably 400,000 or less, and most preferably 300,000 or less.

The diameter of the aggregate is defined as the maximum width of the aggregate. That is, the diameter of the aggregate is the length of the largest straight line connecting two points on the outer edge of the aggregates. Since the aggregates are substantially spherical, the size of the aggregates may be referred to as the diameter of the aggregates for convenience.

The diameter of the aggregate can be controlled by adjusting the number of cells to be seeded in the culture vessel and the culture period. When cultured in a multiwell plate, one cell aggregate is formed in one well. For example, when the number of seeded cells per well is about 5,000 in the case of mouse embryo-derived nephron progenitor cells, an aggregate containing about 100,000 cells after 96 hours is obtained, and when the number of seeded cells per well is about 20,000 in the case of human iPS cell-derived nephron progenitor cells, an aggregate containing about 100,000 to about 300,000 cells after 168 hours is obtained.

Also in the case of culturing using a culture vessel such as a dish or a spinner flask, the diameter of aggregates or the number of cells constituting one aggregate can be controlled by adjusting the seeding density and the number of days of culturing.

The cell aggregates used in the present invention can be of any diameter, for example, 50 µm or more, 100 µm or more. In some cases, the thickness can be 200 µm or more and 500 µm or more. When the cell aggregate is in such a range, the therapeutic effect tends to be high. On the other hand, when the diameter of the cell aggregate is smaller than the inner diameter of the needle of the transplantation device, the aggregate does not break when passing through the needle, which is preferable. Therefore, in general, the diameter is preferably 1,000 µm or less. Preferably, 80% or more of the total number of aggregates is within the desired diameter range (for example, 200 µm or more and 1,000 µm or less), and more preferably, 90% or more of the total number of aggregates is within the desired diameter range (for example, 200 µm or more and 1,000 µm or less).

Such cell aggregates are usually formed, then collected from the medium and concentrated. If necessary, a fractionation process of fractionating the cell aggregates by size may be performed before or after the concentration process. The fractionation can be performed by any known method, for example, by a cell strainer. As a specific example, by passing a medium containing a population of cell aggregates immediately after cell culture through two types of cell strainers (mesh diameter 1,000 µm, 200 µm) of pluriStrainer (registered trademark, pluriSelect), the cell aggregates are fractionated into cell aggregates of 1,000 µm or more on a cell strainer having a mesh diameter of 1,000 µm, cell aggregates of 200 µm or more and 1,000 µm or less on a cell strainer having a mesh diameter of 200 µm, and cell aggregates of 200 µm or less on a filtrate. Thereby, the diameter of the cell aggregate can be made uniform in a desired range.

The collection may be performed by sedimentation of cell aggregates by allowing the medium to stand, or may be performed by centrifugation. After collecting the cell aggregates and removing the excess medium, the cell aggregates may be suspended in an appropriate amount of liquid to obtain a concentrated suspension of cell aggregates.

The liquid for suspending the cell aggregates is not particularly limited as long as the liquid does not adversely affect the cell aggregates, and may be a medium in which a small amount of aggregates are left when the aggregates are collected, a fresh medium, or a liquid other than the medium (for example, physiological saline, buffer solution, or the like). The liquid may contain salts, buffers, serum, vitamins, amino acids, glucose (sugar), electrolytes, antibiotics, and growth factors (compounds, proteins). However, the liquid is preferably substantially free of a cell cycle inhibitor. In other words, it is preferable that the cell cycle inhibitor is not substantially added to the liquid. Here, the liquid "does not substantially contain a cell cycle inhibitor" indicates that the liquid "contains a cell cycle inhibitor to an extent that the cell cycle of the cell is not affected, or does not contain a cell cycle inhibitor at all". Specifically, the concentration of the cell cycle inhibitor in the liquid is preferably less than 3 ng/mL, less than 1 ng/mL, and particularly preferably 0 ng/mL.

The liquid may contain a viscosity modifier. The addition of the viscosity modifier may be before or after suspending the cell aggregates in the liquid. Examples of the viscosity modifier include methylcellulose and gellan gum. The viscosity modifier can be added at a concentration of 0.1% by mass to 1.0% by mass based on the entire solution. Alternatively, the viscosity modifier can be added such that the viscosity of the solution is 0.8 mPa s to 25 mPa·s (JIS Z8803) at 4°C. The viscosity modifier can delay or reduce the contact between the cell aggregates by reducing the sedimentation rate of the cell aggregates in the concentrated suspension of the cell aggregates. Therefore, by containing the viscosity modifier, the effect of inhibiting adhesion between cell aggregates is enhanced.

The liquid may contain lysophospholipid. The lysophospholipid may be added before or after suspending the cell aggregates in the liquid. Examples of the lysophospholipid include lysophosphatidic acid (LPA) and sphingosine-1-phosphate (S1P). The lysophospholipid can be added at a concentration of 1.0 ng/mL to 10.0 mg/mL based on the entire solution. The lysophospholipid can inhibit adhesion between cell aggregates in a concentrated suspension of cell aggregates. Therefore, by containing lysophospholipid, the effect of inhibiting adhesion between cell aggregates is enhanced.

A method for producing a cell aggregate, which is an embodiment of the present invention includes a cooling process of cooling a liquid containing a plurality of cell aggregates, that is, a suspension of cell aggregates, at a temperature lower than the cell culture temperature, to a temperature (hereinafter sometimes referred to as "maintenance temperature" for convenience) which is lower than the cell culture temperature and at which the liquid does not freeze.

As the temperature which is lower than the cell culture temperature and at which the liquid does not freeze, an optimal temperature can be selected as appropriate depending on the type of cells, the use of the cell aggregate, the period during which function maintenance and/or adhesion inhibition are required. The maintenance temperature can be, for example, a temperature which is 10°C or higher, preferably 15°C or higher, and more preferably 20°C or higher lower than the cell culture temperature. The maintenance temperature is generally 36°C or lower, preferably 32°C or lower, 28°C or lower, 24°C or lower, 22°C or lower, and more preferably 16°C or lower. The lower the maintenance temperature, the lower the cell activity and the longer the adhesion inhibition time tends to be. Further, the maintenance temperature is generally higher than 0°C, preferably 2°C or higher, and preferably 4°C or higher. When the maintenance temperature is within this range, partial freezing within the cells is less likely to occur, and cell aggregates with a high cell viability can be obtained. Note that the maintenance temperature may be constant or may be managed within a predetermined temperature range.

Cooling can be performed by maintaining the container containing the suspension of cell aggregates until the maintenance temperature is reached using an incubator, constant temperature room, refrigerator, and the like adjusted to the desired temperature. Alternatively, after completion of the culture, the culture medium may be suctioned off and cooled rapidly by adding new cooled liquid. Cooling may proceed gradually, but it is preferable to complete the cooling after the completion of culture until reaching the maintenance temperature within 6 hours. Cooling can be initiated at a desired time after the formation of cell aggregates, if necessary to inhibit adhesion between cell aggregates. When performing a concentration process and/or a fractionation process, it is preferable to perform a cooling process after these processes.

A cell aggregate maintaining method according to one embodiment of the present invention includes the process of maintaining a liquid containing a plurality of cell aggregates at a maintenance temperature. The maintenance process can be performed by maintaining the container containing the suspension of cell aggregates in an environment at the same temperature as the maintenance temperature achieved in the cooling process. As a result, it was presumed that the adhesion between aggregates was inhibited because (1) intercellular adhesion was directly inhibited by decreasing the gene expression level of intercellular adhesion molecules and/or (2) as a result of inhibition of translation function due to decreased expression level of ribosome-related proteins, expression of intercellular adhesion molecules was inhibited, leading to indirect inhibition of intercellular adhesion.

The period of the maintenance process can be appropriately set as necessary. In general, lower maintenance temperatures tend to allow longer-term maintenance of the number of cell aggregates, cell viability, and cell properties, and for example, the cell aggregates of nephron progenitor cells can be maintained at 16°C for 24 hours or longer and at 4°C for 48 hours or longer. Note that the upper limit time is not particularly limited, and is, for example, 168 hours.

At the end of the maintenance process, the number of cell aggregates can be 40% or more of the number at the beginning of the maintenance process, preferably 60% or more, more preferably 70% or more, and most preferably 85% or more. At the end of the maintenance process, the cell viability of the cell aggregate can be 60% or more, preferably 70% or more, and more preferably 80% or more. The cell viability can be calculated, for example, by converting a cell aggregate into a single cell and determining the percentage of cells that were not stained with trypan blue solution among all cells.

Note that the cooling process and the maintenance process do not necessarily need to be performed by standing. Therefore, the cooling process and the maintenance process can be carried out under conditions where there is vibration to the extent that the cell aggregates do not break, such as on a shaker or during transportation.

The cell aggregates, which is one embodiment of the present invention, can be provided as a cell preparation by being housed in a suitable container. Containers containing cell aggregates include syringes, plastic or glass vials, plastic bottles, plastic bags, centrifuge tubes, and the like. The liquid (preservation solution) filled into the container together with the cell aggregates can be the same liquid as the liquid in which the cell aggregates are suspended, and examples thereof include physiological saline, glucose solution, fresh medium, refrigerated storage medium, and frozen storage medium. The storage solution may contain appropriate additives as necessary. Examples of such additives include low molecular weight compounds such as serum, vitamins, amino acids, glucose (sugar), electrolytes, antibiotics, viscosity modifiers (such as methylcellulose), lysophospholipids, and Rho-associated coiled-coil forming kinase (ROCK) inhibitor; and growth factors (compounds or proteins). The preservation solution is preferably substantially free of cell cycle inhibitors.

The present invention is not limited to the embodiments described above, and it is possible to make various modifications such as design changes based on the knowledge of those skilled in the art, and embodiments with such modifications are also included within the scope of the present invention. Note that any combinations of the above-mentioned components and mutual substitution of the components and expressions of the present invention among methods, devices, systems, and the like are also effective as aspects of the present invention.

### Examples

### 1. Effect of maintenance temperature on mouse embryo-derived nephron progenitor cell aggregates

### <1-1. Morphology of aggregates and cell viability>

Mouse embryo-derived nephron progenitor cells were cultured according to Li Z. et al., Cell Stem Cell. 2016 Oct 06; 19(4): 516-529. 100,000 cells/mL of mouse embryo-derived nephron progenitor cells were seeded in a 96-well plate (PrimeSurface (registered trademark) plate 96U, Sumitomo Bakelite Co., Ltd.) at 50 µL (5,000 cells)/well, and cultured in an incubator at 37°C and 5% CO₂ in a mouse embryo-derived nephron progenitor cell medium (Li et al., supra). On the second day of culture, 100 µL of the medium was overlaid. After 96 hours, it was confirmed by microscopic observation that the mouse embryo-derived nephron progenitor cells had formed aggregates having a diameter of 500 µm to 1,000 µm. The diameter of the cell aggregate was measured using an all-in-one fluorescence microscope (BZ-X710, Keyence Corporation) and analysis application software.

This nephron progenitor cell aggregates was aspirated using a 200 µL tip with a cut off tip, and three cells were placed in each well of a 96-well plate. 100 µL of mouse embryo-derived nephron progenitor cell medium was added to each well of this 96-well plate, and left to stand in an incubator at 4°C, 8°C, 16°C, or 22°C in an atmospheric environment, or left to stand in an incubator at 37°C under 5% CO₂.

After a predetermined period of time had elapsed from the start of standing, the medium in each well was pipetted to confirm the adhesion of the three cell aggregates that were standing at the bottom of the well. Furthermore, the state of adhesion between cell aggregates before and after pipetting was confirmed using an all-in-one fluorescence microscope (BZ-X710, Keyence Corporation). Further, the cell aggregates which were left to stand under each condition were transferred to a 1.5 mL tube and treated with ACCUMAX (trademark, STEMCELL Technologies) for 10 minutes to form single cells. 20 µL each of a cell suspension containing dispersed single cells and a 0.5% trypan blue solution (Nacalai Tesque Inc.) were mixed, and the cell viability was measured using a cell counter (TC20 (trademark), Bio-Rad Laboratories).

Fig. 1 shows the morphology of mouse embryo-derived nephron progenitor cell aggregates which were left to stand at 4°C, 22°C, or 37°C for 24 or 48 hours. Tables 1 and 2 also respectively show the cell viability and adhesion between aggregates after being left to stand at 4°C, 8°C, 16°C, 22°C, or 37°C for the time shown in the tables. In Table 2, the adhesion between aggregates is determined by "A" = no adhesion, "B" = weak adhesion (removed by pipetting), "C" = adhesion (adhesion even after pipetting) (the same applies to Table 5).

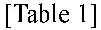

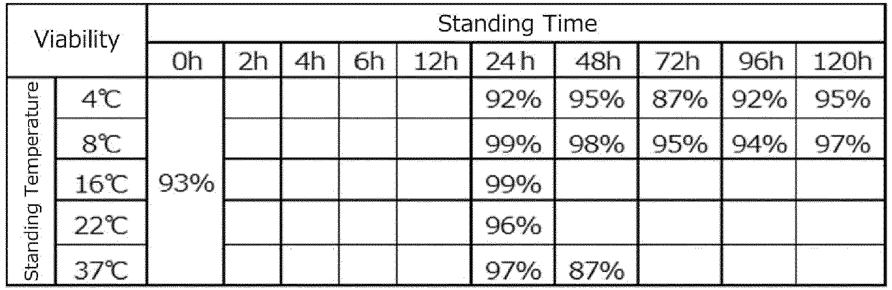

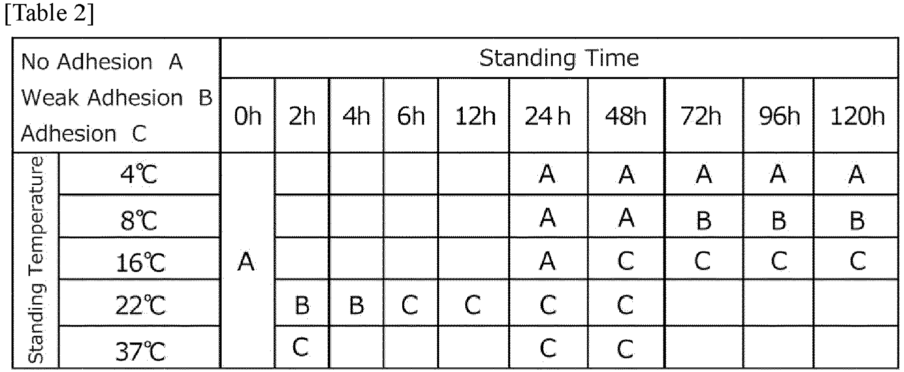

It was shown that when mouse embryo-derived nephron progenitor cell aggregates were maintained at a temperature lower than the normal culture temperature (37°C), adhesion between the aggregates was inhibited while maintaining a high viability. In particular, it was confirmed that when the mouse embryo-derived nephron progenitor cell aggregates were left to stand at 16°C or lower, the aggregates did not adhere to each other for a long time, and a high viability was maintained. Although the tendency for aggregates to adhere to each other gradually increases as the standing temperature approaches 37°C, adhesion inhibition effects can be seen even at 22°C for at least 4 hours, and it was confirmed that adhesion between aggregates could be inhibited for a certain period of time by maintaining the temperature at low temperature.

### <1-2. Physiological functions>

In order to examine whether the mouse embryo-derived nephron progenitor cell aggregates which were left to stand at low temperature maintained the original state of nephron progenitor cells, nephron differentiation potency was confirmed as described by Li et al. (supra).

5,000 mouse embryo-derived nephron progenitor cells were seeded in a 96-well plate and cultured for 96 hours in the same manner as in 1-1 above to form aggregates. Aggregates left to stand at 4°C, 8°C, 16°C, 22°C, or 37°C for 24 or 48 hours, were placed on Transwell (trademark, Corning Inc.), and were cultured in a nephron induction medium (Li et al., supra) for 144 hours at 37°C in a semi-vapor phase under 5% CO₂ conditions. The medium under the Transwell membrane was replaced with 100 µL every 2 days. As a control, nephron induction was performed in the same manner on the mouse embryo-derived nephron progenitor cell aggregates which were normally cultured at 37°C.

Fig. 2 shows the morphology of the mouse embryo-derived nephron progenitor cell aggregates left to stand at 4°C for 48 hours (A) before and (B) after nephron induction, and the morphology of the mouse embryo-derived nephron progenitor cell aggregates obtained by a normal method (C) after nephron induction. Table 3 also shows nephron induction results in mouse embryo-derived nephron progenitor cell aggregates left to stand for 24 hours (or 48 hours) at 4°C, 8°C, 16°C, 22°C, or 37°C. In Table 3, the induction results are expressed as "NG" = no nephron formation, and "OK" = nephron formation (the same applies in Table 6).

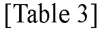

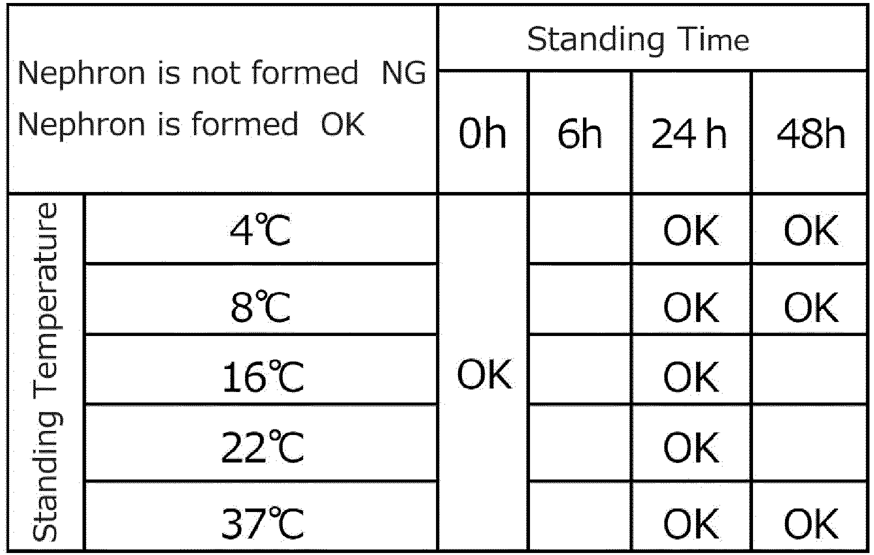

It was confirmed that the mouse embryo-derived nephron progenitor cell aggregates left to stand at 22°C or lower differentiated into nephron tissue, similar to the control. Therefore, it was confirmed that the properties of nephron progenitor cells could be maintained even when mouse embryo-derived nephron progenitor cells were left to stand in an environment lower in temperature than normal culture (37°C, 5% CO₂ environment).

Next, it was examined whether the mouse embryo-derived nephron progenitor cell aggregates left to stand at 4°C would return to the original state in an in vivo environment (37°C).

After the preparation in the same manner as in 1-1 above, the mouse embryo-derived nephron progenitor cell aggregates left to stand at 4°C for 48 hours were transferred to a 1.5 mL tube and treated with ACCUMAX (trademark, STEMCELL Technologies) for 10 minutes to form single cells. 50 µL (5,000 cells) of the suspension adjusted to 100,000 cells/mL was seeded in a 96-well plate, and cultured in a mouse embryo-derived nephron progenitor cell medium at 37°C and 5% CO₂. For comparison, the cells were also cultured at 4°C in an atmospheric environment. At 48 hours of culture, 100 µL of the medium was overlaid, and on the 4th day, 100 µL of the medium was replaced. After 144 hours, the obtained aggregates were placed on Transwell (registered trademark, Corning Inc.), and were cultured in a nephron induction medium for 144 hours in a semi-vapor phase at 37°C in a 5% CO₂ environment. The medium under the Transwell membrane was replaced with 100 µL every 48 hours.

Fig. 3 shows the morphology of repassaged mouse embryo-derived nephron progenitor cells which were cultured for 144 hours at 4°C (A) or 37°C (B) after passage of mouse embryo-derived nephron aggregates maintained at 4°C for 48 hours, and the morphology of (C) after induction of nephron in (B).

It was confirmed that when mouse embryo-derived nephron progenitor cells maintained at 4°C for 48 hours were made into single cells, passaged, and cultured at 37°C, the cells proliferated and aggregates grew, leading to nephron differentiation. Therefore, it was confirmed that when cells of which activity had been decreased by being maintained at 4°C were returned to 37°C, their proliferation potency was restored. On the other hand, it was confirmed that when the cells were cultured at 4°C, cells did not proliferate and aggregates did not grow.

### 2. Effect of maintenance temperature on human iPS cell-derived nephron progenitor cells

### <2-1. Morphology of aggregates and cell viability>

Human iPS cell-derived nephron progenitor cells were prepared and cultured from human iPS cells according to the method for producing nephron progenitor cells (International Publication No. WO2018/216743). Human iPS cell-derived nephron progenitor cells adjusted to 200,000 cells/mL were seeded in a 96-well plate (PrimeSurface (registered trademark) Plate 96U, Sumitomo Bakelite Co., Ltd.) at 100 µL (20,000 cells)/well, and cultured at 37°C and 5% CO₂ in human iPS cell-derived nephron progenitor cell expansion culture medium supplemented with FGF9, CHIR99021, and Y-27632. 100 µL of the medium was replaced at 48 hours, 96 hours, and 144 hours of culture. After 168 hours, it was confirmed that human iPS cell-derived nephron progenitor cells had formed cell aggregates having a diameter of 500 µm to 1,000 µm.

These human iPS cell-derived nephron progenitor cell aggregates were collected using a 200 µL tip with a cut off tip, and five cells were placed in each 1.5 mL tube. These tubes were placed in an incubator at 4°C, 8°C, 16°C, or 22°C in an atmospheric environment, or in an incubator at 37°C in a 5% CO₂ environment.

After a predetermined period of time had elapsed from the start of standing, the culture solution in the 1.5 mL tube was stirred, and the state of adhesion between the human iPS cell-derived nephron progenitor cell aggregates which were left to stand was confirmed. Furthermore, the cells were treated with ACCUMAX (trademark, STEMCELL Technologies) for 10 minutes to form single cells. 20 µL each of a cell suspension containing dispersed single cells and a 0.5% trypan blue solution (Nacalai Tesque Inc.) were mixed, and the cell viability was measured using a cell counter (TC20 (trademark), Bio-Rad Laboratories).

Fig. 4 shows a state immediately after human iPS cell-derived nephron progenitor cell aggregates are transferred to a 1.5 mL tube and left to stand at 4°C for 24 hours and then stirred with the culture solution. It was confirmed that 5 aggregates were maintained without adhesion. In addition, Tables 4 and 5 respectively show the viability of human iPS cell-derived nephron progenitor cell aggregates and the inhibition of aggregate adhesion after being left to stand at 4°C, 8°C, 16°C, 22°C, or 37°C shown in Tables 4 and 5.

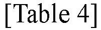

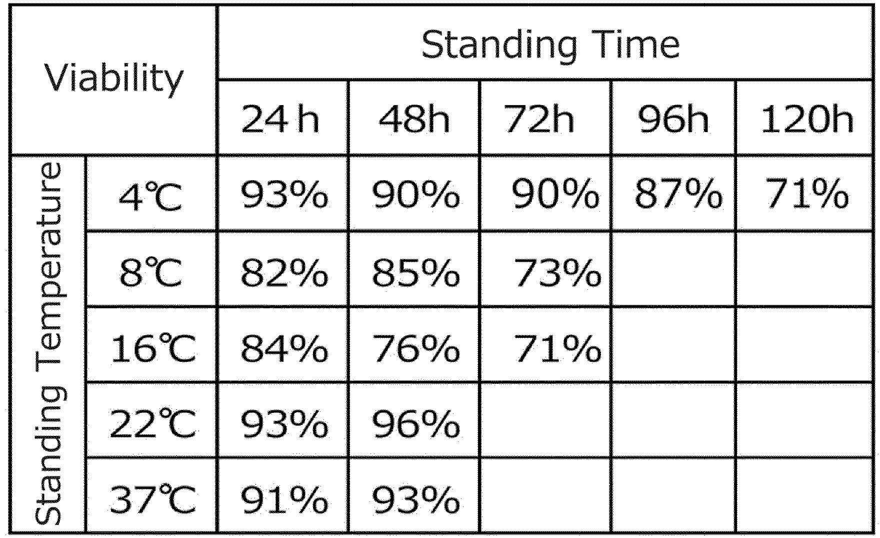

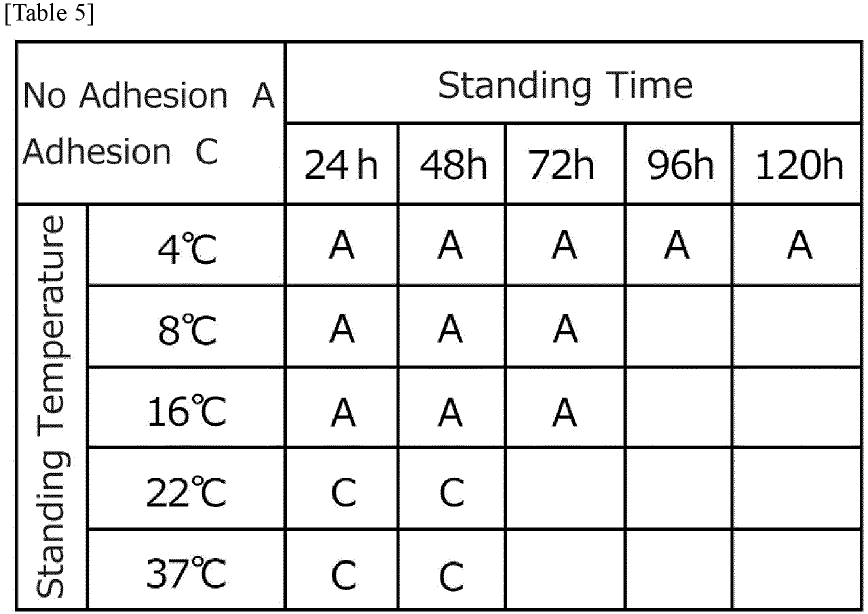

It was confirmed that human iPS cell-derived nephron progenitor cells, similar to mouse embryo-derived n nephron progenitor cells, can inhibit adhesion between aggregates at low temperature while maintaining a high viability.

### <2-2. Physiological functions>

In order to examine whether human iPS cell-derived nephron progenitor cell aggregates left to stand at low temperature maintained the original state of nephron progenitor cells, nephron differentiation potency was confirmed in the same manner as in 1-2 above.

20,000 human iPS cell-derived nephron progenitor cells were seeded in a 96-well plate and cultured for 120 hours in the same manner as in 2-1 above to form aggregates. Aggregates left to stand at 4°C, 8°C, or 16°C for 24 or 48 hours, were placed on Transwell (trademark, Corning Inc.), and were cultured in a nephron induction medium for 144 hours at 37°C in a semi-vapor phase under 5% CO₂ conditions. The medium under the Transwell membrane was replaced with 100 µL every 48 hours. As a control, nephron induction was performed in the same manner on the human iPS cell-derived nephron progenitor cell aggregates which were normally cultured at 37°C. The results are shown in Table 6.

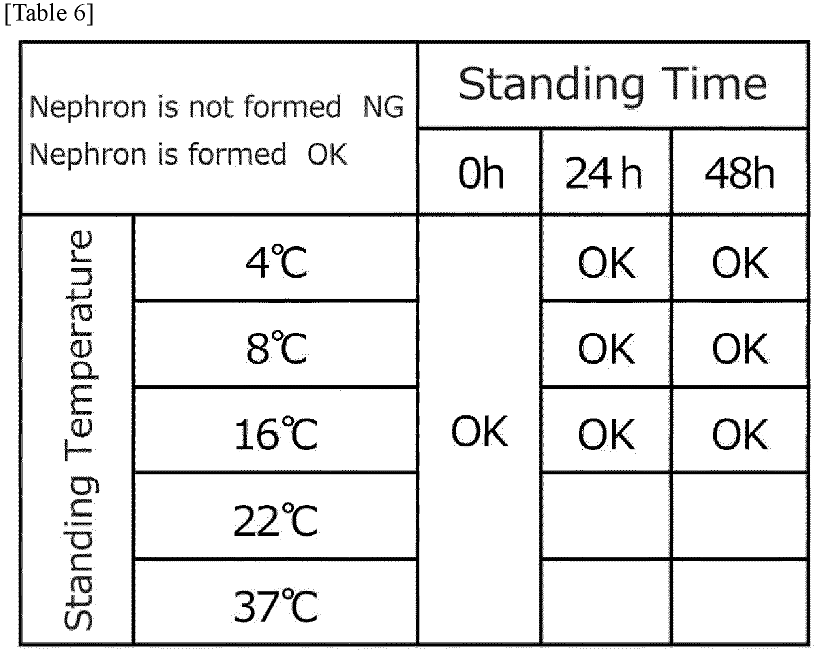

It was confirmed that the human iPS cell-derived nephron progenitor cell aggregates left to stand at 16°C or lower differentiated into nephron tissue, similar to the control. Therefore, it was confirmed that the properties of nephron progenitor cells could be maintained even when human iPS cell-derived nephron progenitor cells left to stand in an environment lower in temperature than normal culture (37°C, 5% CO₂ environment).

Next, it was examined whether the human iPS cell-derived nephron progenitor cell aggregates left to stand at 4°C would return to the original state in an in vivo environment (37°C).

Aggregates were prepared in the same manner as in 2-1 above, and after 48 hours of culture, it was confirmed that human iPS cell-derived nephron progenitor cells had formed cell aggregates having a diameter of 500 µm to 800 µm.

This human iPS cell-derived nephron progenitor cell aggregate was aspirated using a 200 µL tip with a cut off tip, and three cells were placed in each well of a 96-well plate. To each well of this 96-well plate, 100 µL of HypoThermosol (registered trademark, HemaCare), which is a refrigerated storage medium, or a human iPS cell-derived nephron progenitor cell expansion culture medium was added. Those with refrigerated storage medium added were left to stand in a 4°C incubator in an atmospheric environment, and those with human iPS cell-derived nephron progenitor cell expansion culture medium added were left to stand in a 37°C incubator under 5% CO₂ conditions, respectively. After 24 hours, the medium in the well left to stand in a 4°C incubator was replaced with 100 µL of human iPS cell-derived nephron progenitor cell expansion culture medium, and was left to stand in a 37°C incubator under 5% CO₂ for 24 hours.

Fig. 5 shows the morphology of human iPS cell-derived nephron progenitor cell aggregates after each elapsed time. Similarly to 2-1 above, after 24 hours, when the human iPS cell-derived nephron progenitor cell aggregates were left to stand at 37°C, the aggregates adhered to each other, but when the human iPS cell-derived nephron progenitor cell aggregates were left to stand at 4°C, the aggregates were maintained without adhering to each other. Furthermore, when the aggregates left to stand at 4°C and then at 37°C for another 24 hours, the aggregates adhered to each other. Therefore, it was confirmed that when cells of which adhesiveness between aggregates had decreased due to being maintained at 4°C were returned to 37°C, the adhesiveness between aggregates was restored.

Furthermore, among the human iPS cell-derived nephron progenitor cell aggregates described above, aggregates before standing (control), aggregates after being left to stand at 4°C for 24 hours, and some of the aggregates after being left to stand at 4°C for 24 hours and further, after being left to stand at 37°C for 24 hours were sampled, and mRNA was extracted and purified using RNeasy (registered trademark) Mini Kit (QIAGEN). Furthermore, cDNA synthesis was performed using QuantiTect (registered trademark) Reverse Transcription Kit (QIAGEN). Using these cDNAs as templates, the gene expression levels of SRP54, RPL4, and SIX2 were examined by real-time PCR (Thermal Cycler Dice Real Time System I, Takara Bio Inc.).

Fig. 6 shows the measurement results of the gene expression levels of SRP 54, RPL4, and SIX2 in human iPS cell-derived nephron progenitor cell aggregates in each aggregate (relative values with the average of the control group as 1).

The expression levels of SRP54, which is a protein transport-related marker gene, and RPL4, which is a protein synthesis-related marker gene, decreased when the temperature was maintained at 4°C, and the expression levels tended to be restored when the temperature was returned to 37°C. On the other hand, the expression level of SIX2, which is a nephron progenitor cell-related marker gene, was similar in the three test sections. Therefore, it was confirmed that human iPS cell-derived nephron progenitor cell aggregates maintained the properties of nephron progenitor cells when stored at low temperatures, and the properties did not change even after 24 hours of storage.

### <2-3. Adhesion inhibition mechanism between aggregates>

Aggregates were prepared in the same manner as in 2-1 above, and after 48 hours of culture, it was confirmed that human iPS cell-derived nephron progenitor cells had formed cell aggregates having a diameter of 500 µm to 800 µm.

This human iPS cell-derived nephron progenitor cell aggregate was aspirated using a 200 µL tip with a cut off tip, and three cells were placed in each well of a 96-well plate. To each well of this 96-well plate, 100 µL of HypoThermosol (registered trademark, HemaCare), which is a refrigerated storage medium, or a human iPS cell-derived nephron progenitor cell expansion culture medium was added. Those with refrigerated storage medium added were left to stand in a 4°C incubator in an atmospheric environment, and those with human iPS cell-derived nephron progenitor cell expansion culture medium added were left to stand in a 37°C incubator under 5% CO₂ conditions, respectively.

After 48 hours, it was confirmed by pipetting that when the human iPS cell-derived nephron progenitor cell aggregates were left to stand at 37°C, the aggregates adhered to each other, but when the human iPS cell-derived nephron progenitor cell aggregates were left to stand at 4°C, the aggregates were maintained without adhering to each other.

In addition, some of each of the human iPS cell-derived nephron progenitor cell aggregates described above was sampled, and mRNA was extracted and purified using RNeasy (registered trademark) Mini Kit (QIAGEN). For each purified mRNA sample, lead information was obtained using a next-generation sequencer (NovaSeq (registered trademark) 6000, Illumina Inc.). RNA sequence analysis (ENCODE-DCC long read RNA-seq pipeline (2.3.4); https://www.encodeproject.org/pipelines/ENCPL002LPE/) was performed using the obtained read information. Specifically, first, a reference sequence was created and mapped. Next, using the R language (R(3.6.3); https://www.r-project.org/), a two-group comparison (DESeq2(1.26.0)) was performed to detect genes of which expression levels varied between samples.

Next, Gene Ontology (GO) enrichment analysis (GSEA (4.1.0); https://www.gsea-msigdb.org/gsea/index.jsp) was performed using the results of the above RNA sequence analysis. Specifically, the genes of which expression levels varied between samples were analyzed from the viewpoints of cellular components, biological processes, and molecular functions.

Fig. 7 shows GO terms, GO ID, and Adjusted P-values (also referred to as q-values or Q value) of the top 10 gene groups in which the gene expression level was decreased in the sample left to stand at 4°C, compared with the sample left to stand at 37°C, among the gene groups related to the components of the cell. From Fig. 7, it was found that the expression of the gene group related to ribosome (GO ID: 0022625, 0015934, 0005840, 0022627, and 0015935) and the gene group related to intercellular adhesion (GO ID: 0005925 and 0030055) was decreased in the sample left to stand at 4°C as compared with the sample left to stand at 37°C.

Fig. 8 shows GO terms, GO ID, and Adjusted P-values of the top 10 gene groups in which the gene expression level was decreased in the sample left to stand at 4°C, compared with the sample left to stand at 37°C, among the gene groups related to the biological process. As shown in Fig. 8, the top five gene groups were all gene groups related to translation. Therefore, it was found that the expression of translation-related genes was reduced in the samples left to stand at 4°C compared to the samples left to stand at 37°C.

Fig. 9 shows GO terms, GO ID, and Adjusted P-values of the top 10 gene groups in which the gene expression level was decreased in the sample left to stand at 4°C, compared with the sample left to stand at 37°C, among the gene groups related to the molecular functions. From Fig. 9, it was found that the expression level of the gene group related to RNA binding (GO ID: 0003723) was the lowest, and then the expression level of the gene group related to cadherin binding (GO ID: 0045296) was decreased in the sample left to stand at 4°C as compared with the sample left to stand at 37°C. RNA binding is primarily controlled in the process after RNA transcription (for example, translation). In addition, cadherin is one of the intercellular adhesion molecules and contributes to intercellular adhesion. From the result above, it was presumed that when the aggregates were left to stand at low temperature, (1) intercellular adhesion was directly inhibited by decreasing the gene expression level of intercellular adhesion molecules and/or (2) as a result of inhibition of translation function due to decreased expression level of ribosome-related proteins, expression of intercellular adhesion molecules was inhibited, leading to indirect inhibition of intercellular adhesion.

### 3. Effect of maintenance temperature on human iPS cells

The 201B7 strain (Takahashi K., Yamanaka S. et al., Cell. 2007 Nov 30; 131(5): 861-872) was used as the human iPS cell. Human iPS cells adjusted to 10,000 cells/mL were seeded in a 96-well plate (PrimeSurface (registered trademark) Plate 96U, Sumitomo Bakelite Co., Ltd.) at 100 µL (1,000 cells)/well, and cultured with human iPS cell medium (StemFit (registered trademark)), Ajinomoto Co., Inc.) under conditions of 37°C and 5% CO₂. At the start of culture, ROCK inhibitor (Y-27632, Fujifilm Wako Pure Chemical Industries, Ltd.) was added to the medium at a concentration of 10 µM, and after 24 hours, the medium was replaced with 100 µL of human iPS cell medium. After 48 hours, it was confirmed by microscopic observation that the human iPS cells had formed aggregates with a diameter of 100 µm to 400 µm.

This human iPS cell aggregate was aspirated using a 200 µL tip with a cut off tip, and three cells were placed in each well of a 96-well plate. To each well of this 96-well plate, 100 µL of HypoThermosol (registered trademark, HemaCare), which was a refrigerated storage medium, or a human iPS cell medium was added. Those with refrigerated storage medium added were left to stand in a 4°C incubator in an atmospheric environment, and those with human iPS cell medium added were left to stand in a 37°C incubator under 5% CO₂ conditions, respectively. After 24 hours, the medium in the well left to stand in a 4°C incubator was replaced with 100 µL of human iPS cell medium, and was left to stand in a 37°C incubator under 5% CO₂ for 24 hours.

Fig. 10 shows the morphology of human iPS cell aggregates after each elapsed time. After 24 hours, when the human iPS cell aggregates were left to stand at 37°C, the aggregates adhered to each other, but when the human iPS cell aggregates were left to stand at 4°C, the aggregates were maintained without adhering to each other. Furthermore, when the aggregates left to stand at 4°C and then at 37°C for another 24 hours, the aggregates adhered to each other. Accordingly, it was confirmed that human iPS cells, similar to mouse embryo-derived nephron progenitor cells, can inhibit adhesion between aggregates at low temperature. In addition, it was confirmed that when cells of which adhesiveness between aggregates had decreased due to being maintained at 4°C were returned to 37°C, the adhesiveness between aggregates was restored.

### 4. Therapeutic effects using human iPS cell-derived nephron progenitor cells

In the same manner as in 2-1 above, human iPS cell-derived nephron progenitor cells were prepared from human iPS cells and cultured to prepare a human iPS cell-derived nephron progenitor cell aggregate. However, human iPS cell-derived nephron progenitor cells adjusted to 1,000,000 cells/mL were seeded in a 96-well plate (PrimeSurface (registered trademark) Plate 96U, Sumitomo Bakelite Co., Ltd.) at 100 µL (100,000 cells)/well, and cultured for two days at 37°C and 5% CO₂ in human iPS cell-derived nephron progenitor cell expansion culture medium. The diameter of the aggregates was 500 µm to 800 µm.

This human iPS cell-derived nephron progenitor cell aggregates were stored in an atmospheric environment in a refrigerator set at 4°C for 24 hours. When the storage temperature of the place where the human iPS cell-derived nephron progenitor cell aggregates were left to stand in the refrigerator was measured with a thermometer, the result showed 2°C to 4°C. It was confirmed by pipetting that adhesion between aggregates could be inhibited by maintaining the aggregates at low temperature.

These aggregates were administered by transplantation into 6 mice per group of cisplatin-induced acute kidney injury model mice (Li et al., supra) at a total cell amount of 3,000,000 cells per mouse (20 human iPS cell-derived nephron progenitor cell aggregates). Fig. 11 shows a photograph of human iPS cell-derived nephron progenitor cell aggregates (black arrows) immediately after administration under the left renal capsule of a cisplatin-induced acute kidney injury model mouse. As a control, a physiological saline administration group (6 animals) was prepared, which was the same except that approximately 50 µL of physiological saline was administered instead of the human iPS cell-derived nephron progenitor cell aggregates.

Four days after administration, blood urea nitrogen (BUN) values, which were renal function markers, and serum creatinine concentrations were measured, and the results were statistically evaluated using the Student's t test.

The results are shown in Fig. 12. A is the result of the blood BUN value ("BUN"), and B is the result of the serum creatinine concentration ("Cre"). "pre" is the value before administration of human iPS cell-derived nephron progenitor cell aggregates ("NPC") or physiological saline ("saline"), and "post" is the value on the fourth day after administration.

In the group to which human iPS cell-derived nephron progenitor cell aggregates were administered, both the blood BUN value and serum creatinine concentration were significantly lower than in the physiological saline administration group. Therefore, human iPS-derived nephron progenitor cell aggregates stored at 4°C for 24 hours were found to be effective in inhibiting renal function deterioration in cisplatin-induced acute kidney injury model mice.

### 5. Transport test of human iPS cell-derived nephron progenitor cell aggregates

In the same manner as in 4. above, human iPS cell-derived nephron progenitor cells were prepared from human iPS cells and cultured to prepare human iPS cell-derived nephron progenitor cell aggregates. The diameter of the aggregates was 500 µm to 800 µm.

This human iPS cell-derived nephron progenitor cell aggregate (total cell amount of 3,000,000 cells, 20 human iPS cell-derived nephron progenitor cell aggregates) and 25 mL of refrigerated storage medium HypoThermosol (registered trademark, HemaCare) were put into a 25 mL tube and suspended. Furthermore, this 25 mL tube was stored in iP-TEC (registered trademark) Premier BOX-V8.5 (Sampratec Co., Ltd.), which is a cryogenic transport system. Latent heat storage material-5 (Sampratec Co., Ltd.) was placed inside the storage container to maintain the temperature inside the storage container at 5°C to 6°C. After 48 hours from storage, the culture solution in the 25 mL tube was stirred by pipetting, and the state of adhesion between the human iPS cell-derived nephron progenitor cell aggregates which were left to stand was confirmed.

For comparison, a 25 mL tube prepared in the same manner was left to stand in a refrigerator set at 4°C (actual value 2°C to 4°C) instead of being placed in a storage container, and was stored for 48 hours in the same manner as the atmospheric environment.

These human iPS cell-derived nephron progenitor cell aggregates were treated with ACCUMAX (trademark, STEMCELL Technologies) for 10 minutes to form single cells. 20 µL each of a cell suspension containing dispersed single cells and a 0.5% trypan blue solution (Nacalai Tesque Inc.) were mixed, and the cell viability was measured using a cell counter (TC20 (trademark), Bio-Rad Laboratories). In addition, some of the aggregates was sampled, and mRNA was extracted and purified using RNeasy (registered trademark) Mini Kit (QIAGEN). Furthermore, cDNA synthesis was performed using QuantiTect (registered trademark) Reverse Transcription Kit (QIAGEN). Using these cDNAs as templates, the gene expression levels of OSR1 and SIX2 were examined by real-time PCR (Thermal Cycler Dice Real Time System I, Takara Bio Inc.).

As a result, the internal temperature of the storage container of the transport system was maintained at around 6°C for 48 hours, and the cell aggregates were maintained without adhesion. Furthermore, the cell viability of the human iPS cell-derived nephron progenitor cell aggregates stored in the storage container maintained over 90% after 48 hours.

Fig. 13 shows the results of measuring the expression level of a nephron progenitor cell-related marker gene in human iPS cell-derived nephron progenitor cell aggregates before storage and after storage in a storage container (5°C to 6°C) or in a refrigerator (2°C to 4°C) for 48 hours (3 samples for each group) (relative value with the average of the pre-storage group as 1), and the result of a significant difference test by one-way ANOVA. A shows the result of OSR1 and B shows the result of SIX2.

There was no significant difference in the expression levels of OSR1 and SIX2, which are nephron progenitor cell-related marker genes, among the three groups. Therefore, it was confirmed that the properties of human iPS cell-derived nephron progenitor cell aggregates were maintained by the low-temperature storage in a transport system or a refrigerator, and there was no change in the performance of human iPS cell-derived nephron progenitor cell aggregates even after storage for 48 hours.

### 6. Preparation and maintenance of cell aggregates in mass culture system

4,000,000 mouse embryo-derived nephron progenitor cells were seeded in a 100 mL single-use bioreactor (ABLE Inc.), and cultured with stirring at 40 rpm in 100 mL of a mouse embryo-derived nephron progenitor cell medium. 50 mL of the medium was replaced at 48 hours, 96 hours, 120 hours, 144 hours, and 168 hours of culture. After culturing for 192 hours, stirring was stopped to allow cell aggregates to settle, and then the supernatant was removed by suction. After performing resuspension by newly adding 10 mL of fresh medium, the cell aggregates were collected in a 15 mL tube and allowed to settle naturally, to obtain aggregates with a volume of about 2 mL. This was dispensed into four 15 mL tubes, 10 mL of fresh medium was added, and the tubes were left to stand at 4°C or 37°C.

After 24 hours, the medium was resuspended and the presence or absence of adhesion between cell aggregates was confirmed. Furthermore, the cells were treated with ACCUMAX (trademark, STEMCELL Technologies) for 10 minutes to form single cells. 20 µL each of a cell suspension containing dispersed single cells and a 0.5% trypan blue solution (Nacalai Tesque Inc.) were mixed, and the cell viability was measured using a cell counter (TC20 (trademark), Bio-Rad Laboratories).

Fig. 14 shows mouse embryo-derived nephron progenitor cell aggregates (A) immediately after culturing in a 100 mL single use bioreactor and (B) in a state of being transferred to a 15 mL tube and naturally settled. Also shown is the morphology of mouse embryo-derived nephron progenitor cell aggregates immediately after culturing in a bioreactor (0 h, C) and after being left to stand at 4°C for 24 hours (24 h, D). When observed under a microscope, these nephron progenitor cell aggregates had a diameter of about 200 µm to about 1,000 µm.

It was confirmed that mouse embryo-derived nephron progenitor cell aggregates maintained the desired aggregate diameter without adhesion between the aggregates even after being maintained at 4°C for 24 hours. Furthermore, the cell viability was 87%, confirming that a high cell viability was maintained.

On the other hand, it was confirmed that when the aggregates maintained at 37°C for 24 hours, the aggregates adhered to each other and huge aggregates were formed. The cell viability was also as low as 10%, and it was confirmed that the desired aggregate diameter and function could not be maintained.

### 7. Adhesion inhibition effect by additives

4,000,000 mouse embryo-derived nephron progenitor cells were seeded in a 100 mL single-use bioreactor (ABLE Inc.), and cultured with stirring at 40 rpm in 100 mL of a mouse embryo-derived nephron progenitor cell medium (for convenience, it is sometimes referred to as a normal medium or simply a medium). 50 mL of the medium was replaced at 48 hours, 96 hours, 120 hours, 144 hours, and 168 hours of culture. After culturing for 192 hours, stirring was stopped to allow cell aggregates to settle, and then the supernatant was removed by suction. After performing resuspension by newly adding 10 mL of fresh medium or fresh medium supplemented with 0.6% methyl cellulose (Methyl Cellulose 400 (trademark), Fujifilm Wako Pure Chemical Industries, Ltd.), the cell aggregates were collected in a 15 mL tube. This was left to stand at 4°C.

After 24 hours, the medium was resuspended and the presence or absence of adhesion between cell aggregates was confirmed. Furthermore, the cells were treated with ACCUMAX (trademark, STEMCELL Technologies) for 10 minutes to form single cells. 20 µL each of a cell suspension containing dispersed single cells and a 0.5% trypan blue solution (Nacalai Tesque Inc.) were mixed, and the cell viability was measured using a cell counter (TC20 (trademark), Bio-Rad Laboratories).

Fig. 15 shows the state of mouse embryo-derived nephron progenitor cell aggregates 1 minute after suspension in a normal medium (A, left) or a medium supplemented with 0.6% methyl cellulose (A, right), respectively, and transfer to a 15 mL tube. Also shown is the morphology of mouse embryo-derived nephron progenitor cell aggregates (B) immediately after culturing in a bioreactor, and (C) after being left to stand at 4°C for 24 hours in a medium containing 0.6% methylcellulose.

In Fig. 15, a precipitated cell aggregate is confirmed in a normal medium, whereas a sediment is not confirmed in a culture medium supplemented with 0.6% methyl cellulose. Therefore, it was confirmed that by suspending mouse embryo-derived nephron progenitor cell aggregates in a medium supplemented with 0.6% methylcellulose, it was possible to significantly reduce the sedimentation rate of a large amount of concentrated aggregates. It is thought that since the chances of the aggregates settling and coming into contact can be reduced, it becomes possible to further inhibit adhesion between the aggregates.

It was confirmed that when this nephron progenitor cell aggregate was maintained at 4°C for 24 hours in a medium supplemented with 0.6% methylcellulose, the aggregates did not adhere to each other and maintained the desired aggregate diameter. Furthermore, the cell viability was also improved compared to the case of being maintained in the medium shown in 5. above, and it was confirmed that a high viability of 92% was maintained. Therefore, the addition of 0.6% methylcellulose is effective in inhibiting adhesion between aggregates and maintaining cell viability, and is considered to have no adverse effect on cells.

### (Embodiment of invention)

According to a first embodiment of the present invention, there is provided a cell aggregate maintaining method which is a method including a maintenance process of maintaining a liquid containing a plurality of the cell aggregates at a temperature which is lower than a culture temperature of the cells and at which the liquid is not frozen. Accordingly, the effect is achieved in which adhesion and enlargement of aggregates are prevented without using an additive that may adversely affect cells while maintaining the therapeutic effect of cell aggregates.

In the method according to a second embodiment of the present invention, in the first embodiment, further, the temperature is 2°C or higher and 22°C or lower. Accordingly, the effect is achieved in which adhesion and enlargement of cell aggregates such as nephron progenitor cells can be prevented for a longer period of time.

In the method according to a third embodiment of the present invention, in the first or second embodiment, the period of the maintenance process is 2 hours or longer. Accordingly, the effect is achieved in which adhesion and enlargement of cell aggregates such as nephron progenitor cells can be prevented for a longer period of time.

In the method according to a fourth embodiment of the present invention, in the first to third embodiments, the liquid further contains a viscosity modifier. Accordingly, the effect is achieved in which the chance of contact between cell aggregates and the sedimentation rate are reduced, and adhesion and enlargement of cell aggregates are further prevented.

In the method according to a fifth embodiment, in the first to fourth embodiments, further, a diameter of the cell aggregate is 50 µm or more and 1,000 µm or less. Accordingly, the effect is achieved in which the risk of the aggregate breaking when passing through the needle of the implantation device is reduced.

In the method according to a sixth embodiment of the present invention, in the first to fifth embodiments, the cell aggregate is a cell aggregate of undifferentiated cells. Accordingly, the effect is achieved in which cell aggregates that can be used for regenerative medicine treatment of various diseases can be obtained.

In the method according to a seventh embodiment of the present invention, a cell aggregate producing method includes: a culturing process of culturing cells in a culture medium to form a plurality of cell aggregates; and a cooling process of cooling a liquid containing a plurality of the cell aggregates to a temperature which is lower than a culture temperature of the cells and at which the liquid is not frozen. Accordingly, the effect is achieved in which cell aggregates that prevents adhesion and enlargement of aggregates without using an additive that may adversely affect cells while maintaining the therapeutic effect of cell aggregates can be obtained.

In the method according to an eighth embodiment of the present invention, in the seventh embodiment, the method further includes a concentration process of concentrating the cell aggregates into the liquid. Accordingly, the effect is achieved in which the volume of the liquid containing cell aggregates is reduced and cell aggregates suitable for producing, transporting, and using cell preparations are obtained.

In the method according to a ninth embodiment of the present invention, in the seventh or eighth embodiment, the method further includes a fractionation process of fractionating the cell aggregates by diameter. Accordingly, the effect is achieved in which a cell aggregate suitable for transplantation can be selected.

In the method according to a tenth embodiment of the present invention, in the seventh to ninth embodiments, further, the culture medium and the liquid are substantially free of a cell cycle inhibitor. Accordingly, the effect is achieved in which the risk of adverse effects of the cell cycle inhibitor on the cells of the cell aggregate and the living body after transplantation can be reduced, and safety can be improved.

According to an eleventh embodiment of the present invention, there is provided a cell aggregate obtained by the seventh to tenth embodiments. Accordingly, the effect is achieved in which cell characteristics and viability are maintained, and cell aggregates with high therapeutic effects and suitable for use in regenerative medicine can be obtained.

In the cell aggregate according to a twelfth embodiment of the present invention, in the eleventh embodiment, the cell aggregate has a cell viability of 70% or more after maintaining at the temperature for 24 hours. Accordingly, the effect is achieved in which cell aggregates and cell preparations that maintain a high cell viability until the time of use can be produced and transported.

In the cell aggregate according to a thirteenth embodiment of the present invention, in the eleventh or twelfth embodiment, further, the cell is an undifferentiated cell. Accordingly, the effect is achieved in which treatment of various diseases by regenerative medicine becomes possible.

In the cell aggregate according to a fourteenth embodiment of the present invention, in the eleventh to thirteenth embodiments, further, the cell is a nephron progenitor cell, and at least some of the nephron progenitor cells constituting the cell aggregate have differentiation potency. Accordingly, the effect is achieved in which treatment of renal diseases by regenerative medicine becomes possible.

According to a fifteenth embodiment of the present invention, there is provided a cell preparation including the cell aggregate according to the eleventh to fourteenth embodiments. Accordingly, the effect is achieved in which a cell preparation containing high-quality cell aggregates that maintains the therapeutic effect until the time of use is provided.

### Industrial Applicability

The present invention can be utilized for producing and maintaining cell aggregates that can be used in regenerative medicine and the like.

## Claims

1. A cell aggregate maintaining method, comprising:
a maintenance process of maintaining a liquid containing a plurality of the cell aggregates at a temperature which is lower than a culture temperature of the cells and at which the liquid is not frozen.

2. The method according to claim 1, wherein the temperature is 2°C or higher and 22°C or lower.

3. The method according to claim 1 or 2, wherein a period of the maintenance process is two hours or longer.

4. The method according to claim 1 or 2, wherein the liquid further contains a viscosity modifier.

5. The method according to claim 1 or 2, wherein a diameter of the cell aggregate is 50 µm or more and 1,000 µm or less.

6. The method according to claim 1 or 2, wherein the cell aggregate is a cell aggregate of undifferentiated cells.

7. A cell aggregate producing method, comprising:
a culturing process of culturing cells in a culture medium to form a plurality of cell aggregates; and
a cooling process of cooling a liquid containing a plurality of the cell aggregates to a temperature which is lower than a culture temperature of the cells and at which the liquid is not frozen.

8. The method according to claim 7, further comprising a concentration process of concentrating the cell aggregates into the liquid.

9. The method according to claim 7 or 8, further comprising a fractionation process of fractionating the cell aggregates by diameter.

10. The method according to claim 7 or 8, wherein the culture medium and the liquid are substantially free of a cell cycle inhibitor.

11. A cell aggregate obtained by the method according to claim 7 or 8.

12. The cell aggregate according to claim 11, wherein the cell aggregate has a cell viability of 70% or more after maintaining at the temperature for 24 hours.

13. The cell aggregate according to claim 11, wherein the cell is an undifferentiated cell.

14. The cell aggregate according to claim 11, wherein the cell is a nephron progenitor cell, and at least some of the nephron progenitor cells constituting the cell aggregate have differentiation potency.

15. A cell preparation comprising the cell aggregate according to claim 11.
